# EUROPEAN PATENT APPLICATION

(11) **EP 4 736 785 A1**
(43) Date of publication of application: **06.05.2026**
(21) Application number: 25212533.1
(22) Date of filing: 30.10.2025
(51) Int. Cl.: A61B 17/072, A61B 90/90

(54) **SURGICAL INSTRUMENT**

(30) Priority: 31.10.2024 CN 202411544963
(71) Applicant: Hangzhou Mindray Medical Technology Co., Ltd., Hangzhou, Zhejiang 311508 (CN)
(72) Inventor: HUANG, Zhifan, Shenzhen, 518057 (CN); LI, Xin, Shenzhen, 518057 (CN); LI, Mouyuan, Wuhan, 430206 (CN); JIANG, Ping, Shenzhen, 518057 (CN)
(74) Representative: KIPA AB

(57) **Abstract**

Embodiments of the application provide a surgical instrument, the surgical instrument includes a handle assembly, a tube assembly, a jaw assembly, and a staple cartridge assembly. The staple cartridge assembly includes a staple cartridge base and a firing member, and a driving apparatus in the handle assembly drives the firing member to move relative to the staple cartridge base through a transmission mechanism in the tube assembly. Before a patient's tissue is sutured, the transmission mechanism drives the firing member to move from a firing initial position to a firing end position, to complete firing of the staple cartridge assembly. A resistance received by the firing member in a process of travelling for the firing is changed by providing resistance adjustment positions and adjustment members between the firing initial position and the firing end position, such that a driving current of the driving apparatus generates a current characteristic corresponding to each of the resistance adjustment positions, thereby determining a type of the staple cartridge assembly, or determining a driving mode applied by the driving apparatus to the transmission mechanism. The surgical instrument is capable of implementing identification of the staple cartridge assembly safely and reliably and ensuring a suture effect of the patient's tissue.

## Description

### TECHNICAL FIELD

The application relates to the technical field of medical devices, and in particular to a surgical instrument.

### BACKGROUND

During a surgery, a clinical doctor may cut and suture a patient's tissue by using a surgical suture instrument. The surgical suture instrument may include a staple cartridge, and staples in the staple cartridge are deployed into the patient's tissue by a firing driving apparatus, to implement suture. In an actual clinical operation, it needs to select different types of staple cartridges with different staple heights according to characteristics of the patient's tissue. If the type of the staple cartridge cannot be known when the surgical suture instrument performs firing cutting and suture, final cutting and suture effects may be affected.

At present, identification of the staple cartridge is usually achieved by using a smart chip, sensor, wireless identity (ID) identification system or the like on the market. Since implementation of layout of the sensor requires electrical wiring, it results in difficulty of wiring inside a narrow tube. Another important reason is that waterproof and anticorrosion of the wiring cannot be implemented when the wiring passes through moving joints. Once water stains or dirts enter the wiring, it may result in blockage of an electrical circuit and thus may affect identification of the staple cartridge, finally resulting in loss of functions, affecting an effect of the surgery performed by the clinical doctor, and even resulting in a medical accident. Therefore, how to implement identification of the staple cartridge in the surgical suture instrument safely and reliably and ensure the suture effect by using an appropriate driving mode for a driving apparatus of the staple cartridge, has become an urgent problem to be solved.

### SUMMARY

The following is an overview of the subject matter described in detail in the application. This overview is not intended to limit the scope of protection of the claims.

Embodiments of the application provide a surgical instrument, the surgical instrument is capable of implementing identification of a staple cartridge assembly safely and reliably and ensuring the suture effect of the patient's tissue.

According to an aspect, an embodiment of the application provides a surgical instrument, the surgical instrument includes a handle assembly, a tube assembly, a jaw assembly, and a staple cartridge assembly.

The handle assembly includes a driving apparatus and a control apparatus.

An end of the tube assembly is connected to the handle assembly, the tube assembly includes a transmission mechanism connected to the driving apparatus.

Another end of the tube assembly is connected to the jaw assembly, the jaw assembly includes a first jaw and a second jaw, the first jaw is movably connected to the second jaw, the first jaw and the second jaw are capable of moving relatively to each other to implement closure and opening, a mounting cavity is formed between the first jaw and the second jaw.

The staple cartridge assembly is mounted in the mounting cavity, the staple cartridge assembly includes a staple cartridge base and a firing member arranged at the staple cartridge base, the staple cartridge base is provided with a mounting slot where staples are mounted, a firing initial position away from the mounting slot and a firing end position close to the mounting slot, the transmission mechanism is capable of driving the firing member to move from the firing initial position to the firing end position, a first resistance adjustment position and a second resistance adjustment position are provided between the firing initial position and the firing end position, an adjustment member is arranged at each of the first resistance adjustment position and the second resistance adjustment position and is configured to change a resistance received by the firing member when the firing member passes through each of the first resistance adjustment position and the second resistance adjustment position.

The control apparatus is configured to:
acquire a driving current of the driving apparatus, and extract, from the driving current, a first current characteristic corresponding to the first resistance adjustment position and a second current characteristic corresponding to the second resistance adjustment position; and
determine a type of the staple cartridge assembly according to the first current characteristic and the second current characteristic, or determine a driving mode applied by the driving apparatus to the transmission mechanism according to the first current characteristic and the second current characteristic.

In an embodiment of the application, for different types of staple cartridge assemblies, resistances generated to the firing member at the first resistance adjustment position and/or the second resistance adjustment position may be different; each of the first current characteristic and the second current characteristic includes multiple consecutive current values.

The control apparatus configured to determine a type of the staple cartridge assembly according to the first current characteristic and the second current characteristic, includes: the control apparatus configured to:
determine a first characteristic value according to a range within which a current peak value or an average current value in the first current characteristic falls;
determine a second characteristic value according to a range within which a current peak value or an average current value in the second current characteristic falls; and
determine the type of the staple cartridge assembly according to a combination of the first characteristic value and the second characteristic value.

In an embodiment of the application, the adjustment member may be a protrusion arranged on the staple cartridge base.

In an embodiment of the application, the firing member may be provided with a groove matched with the protrusion.

In an embodiment of the application, for different types of staple cartridge assemblies, at least one of the following conditions may be met:
the protrusion at the first resistance adjustment position is different in shape, length or thickness; or
the protrusion at the second resistance adjustment position is different in shape, length or thickness.

In an embodiment of the application, the adjustment member may be a groove arranged in the staple cartridge base, and the firing member is provided with a protrusion matched with the groove.

In an embodiment of the application, for different types of staple cartridge assemblies, at least one of the following conditions may be met:
the protrusion at the first resistance adjustment position is different in shape, length or thickness;
the protrusion at the first resistance adjustment position matched with the groove is different in number;
the protrusion at the second resistance adjustment position is different in shape, length or thickness; or
the protrusion at the second resistance adjustment position matched with the groove is different in number.

In an embodiment of the application, the firing member may include a slider and a cutting knife, one end of the cutting knife is connected to the slider, and the other end of the cutting knife is connected to the transmission mechanism; the groove is arranged in the slider or the cutting knife.

In an embodiment of the application, the firing member may include a slider and a cutting knife, one end of the cutting knife is connected to the slider, and the other end of the cutting knife is connected to the transmission mechanism; the protrusion is arranged on the slider or the cutting knife.

In an embodiment of the application, for different types of staple cartridge assemblies, at least one of the following conditions may be met:
a distance between the first resistance adjustment position and the firing initial position is different;
a distance between the second resistance adjustment position and the firing initial position is different; or
a distance between the first resistance adjustment position and the second resistance adjustment position is different.

In an embodiment of the application, each of the first current characteristic and the second current characteristic may include multiple consecutive current values, the control apparatus configured to determine a type of the staple cartridge assembly according to the first current characteristic and the second current characteristic, includes any one of: the control apparatus configured to:
determine the type of the staple cartridge assembly according to a time interval between a current peak value in the first current characteristic and a current peak value in the second current characteristic; or
determine the type of the staple cartridge assembly according to a first time period corresponding to the firing member starting to move until reaching a current peak value in the first current characteristic, and a second time period corresponding to the firing member starting to move until reaching a current peak value in the second current characteristic.

According to another aspect, an embodiment of the application provides a surgical instrument, the surgical instrument includes a handle assembly, a tube assembly, a jaw assembly, and a staple cartridge assembly.

The handle assembly includes a driving apparatus and a control apparatus.

An end of the tube assembly is connected to the handle assembly, the tube assembly includes a transmission mechanism connected to the driving apparatus.

Another end of the tube assembly is connected to the jaw assembly, the jaw assembly includes a first jaw and a second jaw, the first jaw is movably connected to the second jaw, the first jaw and the second jaw are capable of moving relatively to each other to implement closure and opening, a mounting cavity is formed between the first jaw and the second jaw.

The staple cartridge assembly is mounted in the mounting cavity, the staple cartridge assembly includes a staple cartridge base and a firing member arranged at the staple cartridge base, the staple cartridge base is provided with a mounting slot where staples are mounted, a firing initial position away from the mounting slot and a firing end position close to the mounting slot, the transmission mechanism is capable of driving the firing member to move from the firing initial position to the firing end position, at least one resistance adjustment position is provided between the firing initial position and the firing end position, an adjustment member is arranged at the resistance adjustment position and is configured to change a resistance received by the firing member when the firing member passes through the resistance adjustment position.

The control apparatus is configured to:
acquire a driving current of the driving apparatus, and extract, from the driving current, current characteristic(s) corresponding to the at least one resistance adjustment position; and
determine a type of the staple cartridge assembly according to the current characteristic(s), or determine a driving mode applied by the driving apparatus to the transmission mechanism according to the current characteristic(s).

In an embodiment of the application, for different types of staple cartridge assemblies, the resistance adjustment position may be different in number.

The control apparatus configured to determine a type of the staple cartridge assembly according to the current characteristic(s), includes: the control apparatus configured to:
determine the type of the staple cartridge assembly according to the number of the current characteristic(s).

According to another aspect, an embodiment of the application provides a surgical instrument, the surgical instrument includes a handle assembly, a tube assembly, a jaw assembly, and a staple cartridge assembly.

The handle assembly includes a driving apparatus and a control apparatus.

An end of the tube assembly is connected to the handle assembly, the tube assembly includes a transmission mechanism connected to the driving apparatus.

Another end of the tube assembly is connected to the jaw assembly, the jaw assembly includes a first jaw and a second jaw, the first jaw is movably connected to the second jaw, the first jaw and the second jaw are capable of moving relatively to each other to implement closure and opening, a mounting cavity is formed between the first jaw and the second jaw.

The staple cartridge assembly is mounted in the mounting cavity, the staple cartridge assembly includes a staple cartridge base and a firing member arranged at the staple cartridge base, the staple cartridge base is provided with a mounting slot where staples are mounted, a firing initial position away from the mounting slot and a firing end position close to the mounting slot, the transmission mechanism is capable of driving the firing member to move from the firing initial position to the firing end position, a first resistance adjustment position and a second resistance adjustment position are provided between the firing initial position and the firing end position, an adjustment member is arranged at each of the first resistance adjustment position and the second resistance adjustment position and is configured to change a resistance received by the transmission mechanism when the transmission mechanism passes through each of the first resistance adjustment position and the second resistance adjustment position.

The control apparatus is configured to:
acquire a driving current of the driving apparatus, and extract, from the driving current, a first current characteristic corresponding to the first resistance adjustment position and a second current characteristic corresponding to the second resistance adjustment position; and
determine a type of the staple cartridge assembly according to the first current characteristic and the second current characteristic, or determine a driving mode applied by the driving apparatus to the transmission mechanism according to the first current characteristic and the second current characteristic.

According to another aspect, an embodiment of the application provides a surgical instrument, the surgical instrument includes a handle assembly, a tube assembly, a jaw assembly, and a staple cartridge assembly.

The handle assembly includes a driving apparatus and a control apparatus.

An end of the tube assembly is connected to the handle assembly, the tube assembly includes a transmission mechanism connected to the driving apparatus.

Another end of the tube assembly is connected to the jaw assembly, the jaw assembly includes a first jaw and a second jaw, the first jaw is movably connected to the second jaw, the first jaw and the second jaw are capable of moving relatively to each other to implement closure and opening, a mounting cavity is formed between the first jaw and the second jaw.

The staple cartridge assembly is mounted in the mounting cavity, the staple cartridge assembly includes a staple cartridge base and a firing member arranged at the staple cartridge base, the staple cartridge base is provided with a mounting slot where staples are mounted, a firing initial position away from the mounting slot and a firing end position close to the mounting slot, the transmission mechanism is capable of driving the firing member to move from the firing initial position to the firing end position, at least one resistance adjustment position is provided between the firing initial position and the firing end position, an adjustment member is arranged at the resistance adjustment position and is configured to change a resistance received by the transmission mechanism when the transmission mechanism passes through the resistance adjustment position.

The control apparatus is configured to:
acquire a driving current of the driving apparatus, and extract, from the driving current, current characteristic(s) corresponding to the at least one resistance adjustment position; and
determine a type of the staple cartridge assembly according to the current characteristic(s), or determine a driving mode applied by the driving apparatus to the transmission mechanism according to the current characteristic(s).

The embodiments of the application include at least the following advantageous effects.

On one hand, a surgical instrument provided in an embodiment of the application includes a handle assembly, a tube assembly, a jaw assembly and a staple cartridge assembly, here the handle assembly, the tube assembly and the jaw assembly are sequentially connected, and the staple cartridge assembly is mounted in a mounting cavity formed between a first jaw and a second jaw of the jaw assembly. The staple cartridge assembly includes a staple cartridge base and a firing member, and after the staple cartridge assembly is mounted in the mounting cavity, the firing member is abutted and connected with a transmission mechanism in the tube assembly, and a driving apparatus in the handle assembly drives the firing member to move relative to the staple cartridge base through the transmission mechanism in the tube assembly. Before the patient's tissue is sutured, the transmission mechanism drives the firing member to move from a firing initial position away from a mounting slot where staples are mounted to a firing end position close to the mounting slot, to complete firing of the staple cartridge assembly. Two resistance adjustment positions are provided between the firing initial position and the firing end position, and adjustment members are arranged at the two resistance adjustment positions respectively, to change resistances received by the firing member when the firing member passes through the two resistance adjustment positions in a process of travelling for the firing, such that a driving current of the driving apparatus generates current characteristics corresponding to the two resistance adjustment positions respectively. Then, a driving current of the driving apparatus is acquired by a control apparatus, and current characteristics corresponding to the two resistance adjustment positions respectively are extracted from the driving current by the control apparatus; finally, a type of the staple cartridge assembly is determined according to a difference between the current characteristics corresponding to the two resistance adjustment positions respectively, or a driving mode applied by the driving apparatus to the transmission mechanism is determined according to the difference between the current characteristics corresponding to the two resistance adjustment positions respectively. In this way, it is unnecessary to provide electrical components and electrical wiring on the staple cartridge assembly, to avoid abnormal identification of the staple cartridge assembly due to blockage of the electrical circuit, and avoid driving the driving apparatus of the staple cartridge assembly by using an inappropriate driving mode, such that it may implement identification of the staple cartridge assembly safely and reliably and ensure the suture effect of the patient's tissue.

On the other hand, according to a surgical instrument provided in an embodiment of the application, at least one resistance adjustment position is provided between a firing initial position and a firing end position, and an adjustment member is arranged at the resistance adjustment position, to change a resistance received by a firing member when the firing member passes through the resistance adjustment position in a process of travelling for the firing, such that a driving current of a driving apparatus generates current characteristic(s) corresponding to the at least one resistance adjustment position. Then, a driving current of the driving apparatus is acquired by a control apparatus, and current characteristic(s) corresponding to the at least one resistance adjustment position are extracted from the driving current by the control apparatus; finally, a type of a staple cartridge assembly is determined according to a difference between the current characteristic(s) corresponding to the at least one resistance adjustment position, or a driving mode applied by the driving apparatus to a transmission mechanism is determined according to the difference between the current characteristic(s) corresponding to the at least one resistance adjustment position. In this way, it is unnecessary to provide electrical components and electrical wiring on the staple cartridge assembly, to avoid abnormal identification of the staple cartridge assembly due to blockage of the electrical circuit, and avoid driving the driving apparatus of the staple cartridge assembly by using an inappropriate driving mode, such that it may implement identification of the staple cartridge assembly safely and reliably and ensure the suture effect of the patient's tissue.

On the other hand, according to a surgical instrument provided in an embodiment of the application, two resistance adjustment positions are provided between a firing initial position and a firing end position, and adjustment members are arranged at the two resistance adjustment positions respectively, to change resistances received by a transmission mechanism when the transmission mechanism passes through the two resistance adjustment positions in a process of travelling for the firing, such that a driving current of a driving apparatus generates current characteristics corresponding to the two resistance adjustment positions respectively. Then, a driving current of the driving apparatus is acquired by a control apparatus, and current characteristics corresponding to the two resistance adjustment positions respectively are extracted from the driving current by the control apparatus; finally, a type of a staple cartridge assembly is determined according to a difference between the current characteristics corresponding to the two resistance adjustment positions respectively, or a driving mode applied by the driving apparatus to the transmission mechanism is determined according to the difference between the current characteristics corresponding to the two resistance adjustment positions respectively. In this way, it is unnecessary to provide electrical components and electrical wiring on the staple cartridge assembly, to avoid abnormal identification of the staple cartridge assembly due to blockage of the electrical circuit, and avoid driving the driving apparatus of the staple cartridge assembly by using an inappropriate driving mode, such that it may implement identification of the staple cartridge assembly safely and reliably and ensure the suture effect of the patient's tissue.

On the other hand, according to a surgical instrument provided in an embodiment of the application, at least one resistance adjustment position is provided between a firing initial position and a firing end position, and an adjustment member is arranged at the resistance adjustment position, to change a resistance received by a transmission mechanism when the transmission mechanism passes through the resistance adjustment position in a process of travelling for the firing, such that a driving current of a driving apparatus generates current characteristic(s) corresponding to the at least one resistance adjustment position. Then, a driving current of the driving apparatus is acquired by a control apparatus, and current characteristic(s) corresponding to the at least one resistance adjustment position are extracted from the driving current by the control apparatus; finally, a type of a staple cartridge assembly is determined according to a difference between the current characteristic(s) corresponding to the at least one resistance adjustment position, or a driving mode applied by the driving apparatus to the transmission mechanism is determined according to the difference between the current characteristic(s) corresponding to the at least one resistance adjustment position. In this way, it is unnecessary to provide electrical components and electrical wiring on the staple cartridge assembly, to avoid abnormal identification of the staple cartridge assembly due to blockage of the electrical circuit, and avoid driving the driving apparatus of the staple cartridge assembly by using an inappropriate driving mode, such that it may implement identification of the staple cartridge assembly safely and reliably and ensure the suture effect of the patient's tissue.

Other features and advantages of the application will be set forth in the following description, part of which will become apparent from the description, or will be learned by implementation of the application.

### BRIEF DESCRIPTION OF THE DRAWINGS

The drawings are intended to provide a further understanding of technical solutions of the application, constitute a part of the description and explain the technical solutions of the application together with the embodiments of the application, and do not constitute a limitation on the technical solutions of the application.
FIG. 1 is a schematic view of an overall appearance of a surgical instrument provided in an embodiment of the application.
FIG. 2 is a cross-sectional view of a transmission mechanism, a jaw assembly and a staple cartridge assembly of a surgical instrument provided in an embodiment of the application.
FIG. 3 is a top view of a staple cartridge assembly of a surgical instrument provided in an embodiment of the application.
FIG. 4 is a flowchart of an operation control method performed by a control apparatus of a surgical instrument provided in an embodiment of the application.
FIG. 5 is a flowchart of an operation control method performed by a control apparatus of a surgical instrument provided in another embodiment of the application.
FIG. 6A to FIG. 6I are schematic curve views of a driving current of a driving apparatus acquired by a control apparatus of a surgical instrument provided in an embodiment of the application.
FIG. 7 is a flowchart of an operation control method performed by a control apparatus of a surgical instrument provided in yet another embodiment of the application.
FIG. 8 is a cross-sectional view of a staple cartridge base provided with a protrusion of a surgical instrument provided in an embodiment of the application.
FIG. 9 is a top view of a slider of a surgical instrument provided in an embodiment of the application.
FIG. 10 is a schematic perspective view of a slider of a surgical instrument provided in an embodiment of the application.
FIG. 11 is a schematic curve view of a driving current of a driving apparatus provided in another embodiment of the application.
FIG. 12 is a schematic curve view of a driving current of a driving apparatus provided in yet another embodiment of the application.

### DETAILED DESCRIPTION

The application will be further described below with reference to the drawings and specific embodiments. The described embodiments should not be considered as limitation of the application, and all other embodiments obtained by those of ordinary skill in the art without paying any creative work fall within the scope of protection of the application.

In the following descriptions, reference is made to "some embodiments" which describes a subset of all possible embodiments; however, it may be understood that "some embodiments" may be the same or different subsets of all possible embodiments, and may be combined with each other without conflict.

Unless otherwise defined, all technical and scientific terms used here have the same meaning as usually understood by technicians in the technical field to which the application belongs. The terms used here are for the purpose of describing the embodiments of the application only, and are not intended to limit the application.

At present, a surgical instrument provided with a staple cartridge assembly may fire and drive the staple cartridge assembly through a driving apparatus, to suture a patient's tissue; however, in an actual clinical operation, it needs to select different types of staple cartridges with different staple heights according to characteristics of the patient's tissue. Therefore, in a process of firing the staple cartridge assembly, identification of the staple cartridge assembly is implemented and the driving apparatus of the staple cartridge assembly is driven by using an appropriate driving mode, which may ensure a suture effect of the patient's tissue.

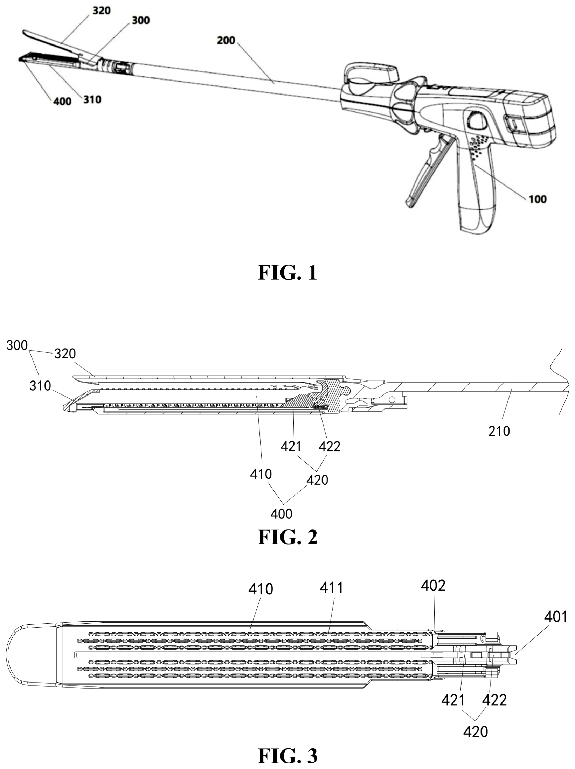

A schematic view of an overall appearance of a surgical instrument provided in an embodiment of the application is shown in FIG. 1. With reference to FIG. 1, the surgical instrument includes a handle assembly 100, a tube assembly 200, a jaw assembly 300, and a staple cartridge assembly 400.

The handle assembly 100 includes a driving apparatus and a control apparatus. Specifically, the driving apparatus and the control apparatus are arranged inside a housing of the handle assembly 100, and thus the driving apparatus and the control apparatus are not shown in FIG. 1.

An end of the tube assembly 200 is connected to the handle assembly 100, the tube assembly 200 includes a transmission mechanism 210 connected to the driving apparatus. Specifically, the transmission mechanism 210 is arranged inside the tube assembly 200, and thus the transmission mechanism 210 is not shown in FIG. 1.

Another end of the tube assembly 200 is connected to the jaw assembly 300, the jaw assembly 300 includes a first jaw 310 and a second jaw 320, the first jaw 310 is movably connected to the second jaw 320, the first jaw 310 and the second jaw 320 are capable of moving relatively to each other to implement closure and opening, a mounting cavity is formed between the first jaw 310 and the second jaw 320. The first jaw 310 and the second jaw 320 shown in FIG. 1 are in an open state, and the first jaw 310 and the second jaw 320 shown in FIG. 2 are in a closed state.

The staple cartridge assembly 400 is mounted in the mounting cavity. As shown in FIG. 2 and FIG. 3, the staple cartridge assembly 400 includes a staple cartridge base 410 and a firing member 420, the firing member 420 is arranged at the staple cartridge base 410, the staple cartridge base 410 is provided with a mounting slot 411 where staples are mounted, a firing initial position 401 away from the mounting slot 411 and a firing end position 402 close to the mounting slot 411. With reference to FIG. 2, after the staple cartridge assembly 400 is mounted into the mounting cavity in the jaw assembly 300, the transmission mechanism 210 is cooperatively connected to the firing member 420 of the staple cartridge assembly 400, such that the transmission mechanism 210 is capable of driving the firing member 420 to move from the firing initial position 401 to the firing end position 402, to complete a process of firing the staple cartridge assembly 400.

A first resistance adjustment position and a second resistance adjustment position are provided between the firing initial position 401 and the firing end position 402, and an adjustment member is arranged at each of the first resistance adjustment position and the second resistance adjustment position and is configured to change a resistance received by the firing member 420 when the firing member 420 passes through each of the first resistance adjustment position and the second resistance adjustment position. It may be understood that in the process of firing the staple cartridge assembly 400, that is, in a process of the transmission mechanism 210 driving the firing member 420 to move from the firing initial position 401 to the firing end position 402, the firing member 420 may pass through the first resistance adjustment position and the second resistance adjustment position, and adjustment members arranged at the two resistance adjustment positions respectively may change resistances received by the firing member 420 when the firing member 420 passes through the two resistance adjustment positions respectively. The resistance encountered by the firing member 420 in the firing process may be represented by a variation of a driving current of the driving apparatus, such that corresponding current characteristics are present in the driving current of the driving apparatus.

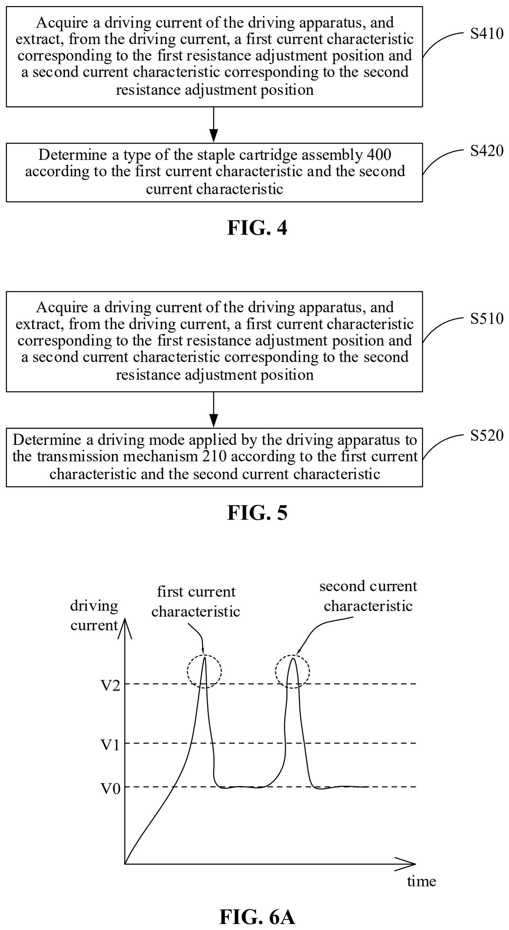

In an embodiment, the control apparatus is configured to perform an operation control method shown in FIG. 4, and the operation control method includes, but is not limited to the following operations S410 and S420.

In operation S410, a driving current of the driving apparatus is acquired, and a first current characteristic corresponding to the first resistance adjustment position and a second current characteristic corresponding to the second resistance adjustment position are extracted from the driving current.

In operation S420, a type of the staple cartridge assembly 400 is determined according to the first current characteristic and the second current characteristic.

In an embodiment, the control apparatus is configured to perform an operation control method shown in FIG. 5, and the operation control method includes, but is not limited to the following operations S510 and S520.

In operation S510, a driving current of the driving apparatus is acquired, and a first current characteristic corresponding to the first resistance adjustment position and a second current characteristic corresponding to the second resistance adjustment position are extracted from the driving current.

In operation S520, a driving mode applied by the driving apparatus to the transmission mechanism 210 is determined according to the first current characteristic and the second current characteristic.

It may be understood that the driving current of the driving apparatus should be a relatively stable current with little fluctuation when the firing member 420 does not encounter any adjustment member, and at this time, the driving apparatus only needs to overcome a friction force between the firing member 420 and the staple cartridge base 410 to drive the firing member 420. However, adjustment members arranged at the first resistance adjustment position and the second resistance adjustment position respectively may increase resistances received by the firing member 420 when the firing member 420 passes through the adjustment members at the first resistance adjustment position and the second resistance adjustment position respectively, and the driving apparatus needs a greater driving current to generate a torque enough to drive the transmission mechanism 210, and then the transmission mechanism 210 drives the firing member 420 to go away from the first resistance adjustment position and the second resistance adjustment position to continue moving forward.

In the operation S410 and the operation S510, after the driving current of the driving apparatus is acquired, the first current characteristic and the second current characteristic that are greater than currents at other positions may be extracted from the driving current of the driving apparatus. Exemplarily, assuming that the driving current of the driving apparatus usually fluctuates around 0.5A when the firing member 420 does not encounter any adjustment member; and when the firing member 420 passes through the adjustment members, it needs to overcome resistances brought by the adjustment members, the driving current of the driving apparatus may increase to be greater than 0.8A, and then two convex regions may be present in a curve of the driving current of the driving apparatus and are the first current characteristic corresponding to the first resistance adjustment position and the second current characteristic corresponding to the second resistance adjustment position respectively, and at this time, each of the first current characteristic and the second current characteristic is formed of multiple consecutive current values greater than 0.8A.

In the operation S420, the type of the staple cartridge assembly 400 is determined according to a difference between the current characteristics corresponding to the two resistance adjustment positions respectively. In this way, it is unnecessary to provide electrical components and electrical wiring on the staple cartridge assembly 400, to avoid abnormal identification of the staple cartridge assembly 400 due to blockage of the electrical circuit, such that it may implement identification of the staple cartridge assembly 400 safely and reliably.

In the operation S520, the driving mode applied by the driving apparatus to the transmission mechanism 210 is determined according to the difference between the current characteristics corresponding to the two resistance adjustment positions respectively, to avoid driving the driving apparatus of the staple cartridge assembly 400 by using an inappropriate driving mode, such that it may ensure the suture effect of the patient's tissue.

It may be understood that different driving modes applied by the driving apparatus to the transmission mechanism 210 may be represented by a fact that the control apparatus provides different amplitudes of driving voltages, different frequencies of driving voltages or different magnitudes of driving currents to the driving apparatus, such that a torque output by the driving apparatus to the transmission mechanism 210 is different, or a speed at which the driving apparatus drives the transmission mechanism 210 to move is different.

In an embodiment, for different types of staple cartridge assemblies 400, resistances generated to the firing member 420 at the first resistance adjustment position and/or the second resistance adjustment position are different.

In this embodiment, the resistances generated to the firing member 420 are different, and current characteristics that may be extracted from the driving current of the driving apparatus are also different naturally. As described previously, each of the first current characteristic and the second current characteristic includes multiple consecutive current values. Exemplarily, if the adjustment member at the resistance adjustment position generates a small resistance to the firing member 420, multiple current values in a corresponding current characteristic are greater than 0.8A but less than 1.2A; if the adjustment member at the resistance adjustment position generates a large resistance to the firing member 420, multiple current values in a corresponding current characteristic are greater than 1.2A.

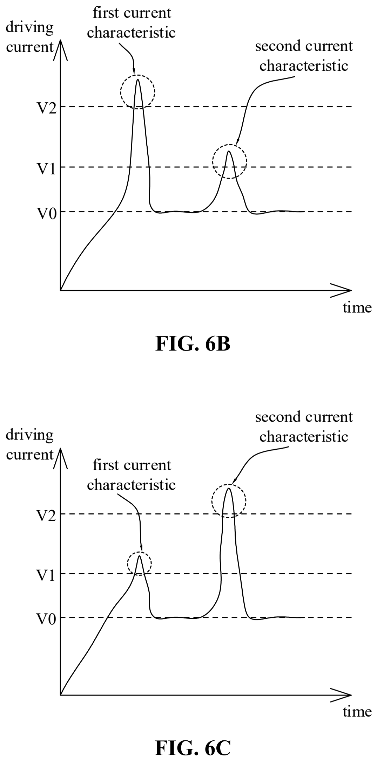

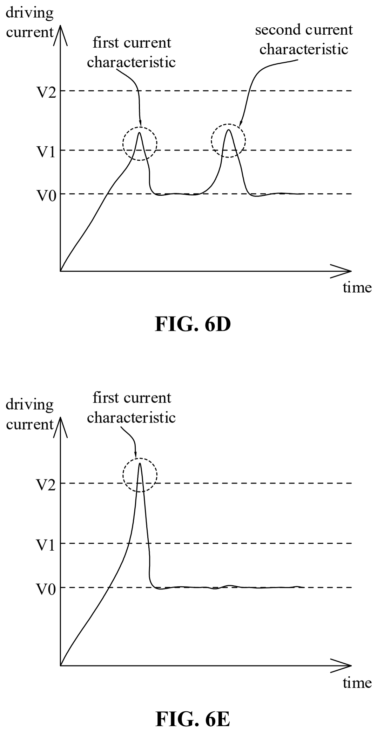

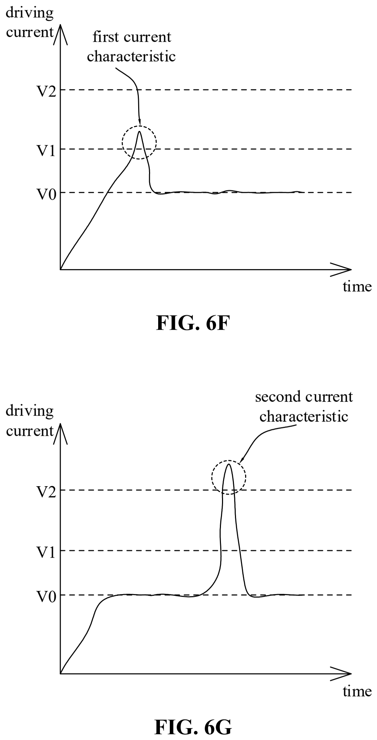

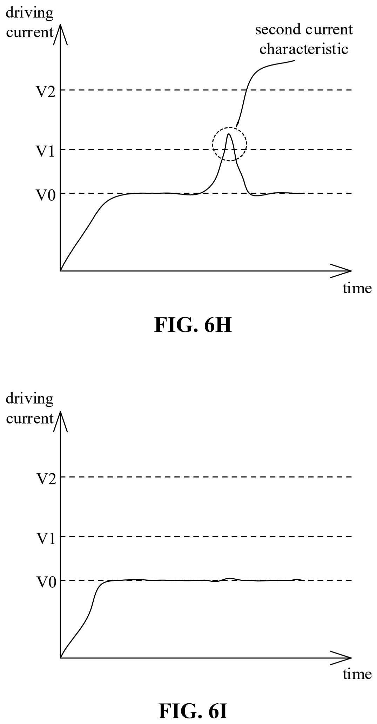

Further, in this embodiment, schematic curve views of the driving current of the driving apparatus that may be acquired are shown in FIG. 6A to FIG. 6I, here V0 may represent 0.5A, V1 may represent 0.8A, and V2 may represent 1.2A. Therefore, FIG. 6A shows that each of the first current characteristic and the second current characteristic extracted from the driving current includes multiple consecutive current values greater than 1.2A, such that it may be determined that the staple cartridge assembly 400 is provided with an adjustment member that generates a large resistance to the firing member 420 at each of the first resistance adjustment position and the second resistance adjustment position. FIG. 6B shows that the first current characteristic extracted from the driving current includes multiple consecutive current values greater than 1.2A, and the second current characteristic extracted from the driving current includes multiple consecutive current values greater than 0.8A and less than 1.2A, such that it may be determined that the staple cartridge assembly 400 is provided with an adjustment member that generates a large resistance to the firing member 420 at the first resistance adjustment position, and the staple cartridge assembly 400 is provided with an adjustment member that generates a small resistance to the firing member 420 at the second resistance adjustment position. FIG. 6C shows that the first current characteristic extracted from the driving current includes multiple consecutive current values greater than 0.8A and less than 1.2A, and the second current characteristic extracted from the driving current includes multiple consecutive current values greater than 1.2A, such that it may be determined that the staple cartridge assembly 400 is provided with an adjustment member that generates a small resistance to the firing member 420 at the first resistance adjustment position, and the staple cartridge assembly 400 is provided with an adjustment member that generates a large resistance to the firing member 420 at the second resistance adjustment position. FIG. 6D shows that each of the first current characteristic and the second current characteristic extracted from the driving current includes multiple consecutive current values greater than 0.8A and less than 1.2A, such that it may be determined that the staple cartridge assembly 400 is provided with an adjustment member that generates a small resistance to the firing member 420 at each of the first resistance adjustment position and the second resistance adjustment position. FIG. 6E shows that the first current characteristic extracted from the driving current includes multiple consecutive current values greater than 1.2A, and the second current characteristic extracted from the driving current does not include any current value greater than 0.8A, such that it may be determined that the staple cartridge assembly 400 is provided with an adjustment member that generates a large resistance to the firing member 420 at the first resistance adjustment position, and the staple cartridge assembly 400 is not provided with a resistance adjustment member at the second resistance adjustment position. FIG. 6F shows that the first current characteristic extracted from the driving current includes multiple consecutive current values greater than 0.8A and less than 1.2A, and the second current characteristic extracted from the driving current does not include any current value greater than 0.8A, such that it may be determined that the staple cartridge assembly 400 is provided with an adjustment member that generates a small resistance to the firing member 420 at the first resistance adjustment position, and the staple cartridge assembly 400 is not provided with a resistance adjustment member at the second resistance adjustment position. FIG. 6G shows that the first current characteristic extracted from the driving current does not include any current value greater than 0.8A, and the second current characteristic extracted from the driving current includes multiple consecutive current values greater than 1.2A, such that it may be determined that the staple cartridge assembly 400 is not provided with a resistance adjustment member at the first resistance adjustment position, and the staple cartridge assembly 400 is provided with an adjustment member that generates a large resistance to the firing member 420 at the second resistance adjustment position. FIG. 6H shows that the first current characteristic extracted from the driving current does not include any current value greater than 0.8A, and the second current characteristic extracted from the driving current includes multiple consecutive current values greater than 0.8A and less than 1.2A, such that it may be determined that the staple cartridge assembly 400 is not provided with a resistance adjustment member at the first resistance adjustment position, and the staple cartridge assembly 400 is provided with an adjustment member that generates a small resistance to the firing member 420 at the second resistance adjustment position. FIG. 6I shows that each of the first current characteristic and the second current characteristic extracted from the driving current does not include any current value greater than 0.8A, such that it may be determined that the staple cartridge assembly 400 is not provided with a resistance adjustment member at each of the first resistance adjustment position and the second resistance adjustment position, or it may be determined that the staple cartridge assembly 400 is not placed in the mounting cavity of the jaw assembly 300.

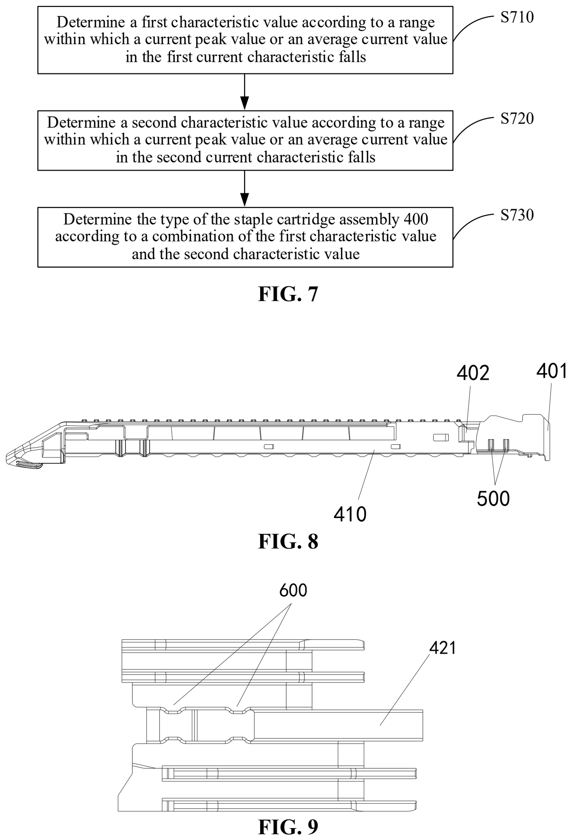

In an embodiment, as shown in FIG. 7, the operation of determining the type of the staple cartridge assembly 400 according to the first current characteristic and the second current characteristic from the operation S420 includes the following operations S710 to S730.

In operation S710, a first characteristic value is determined according to a range within which a current peak value or an average current value in the first current characteristic falls.

In this operation, if the current peak value or the average current value in the first current characteristic falls within a range greater than 1.2A, the first characteristic value is H; if the current peak value or the average current value in the first current characteristic falls within a range of 0.8A to 1.2A, the first characteristic value is L; if the current peak value or the average current value in the first current characteristic falls within a range less than 0.8A, the first characteristic value is /.

In operation S720, a second characteristic value is determined according to a range within which a current peak value or an average current value in the second current characteristic falls.

In this operation, if the current peak value or the average current value in the second current characteristic falls within a range greater than 1.2A, the second characteristic value is H; if the current peak value or the average current value in the second current characteristic falls within a range of 0.8A to 1.2A, the second characteristic value is L; if the current peak value or the average current value in the second current characteristic falls within a range less than 0.8A, the second characteristic value is /.

In operation S730, the type of the staple cartridge assembly 400 is determined according to a combination of the first characteristic value and the second characteristic value.

In this embodiment, there are a total of nine combinations of the first characteristic value and the second characteristic value, which are HH, HL, LH, LL, H/, L/, /H, /L and // as shown in the following Table 1 respectively, and correspond to the above FIG. 6A to FIG. 6I respectively. Here a type of the staple cartridge assembly corresponding to the combination HH is a black staple cartridge assembly, a type of the staple cartridge assembly corresponding to the combination HL is a green staple cartridge assembly, a type of the staple cartridge assembly corresponding to the combination LH is a blue staple cartridge assembly, a type of the staple cartridge assembly corresponding to the combination LL is a white staple cartridge assembly, a type of the staple cartridge assembly corresponding to the combination H/ is a gold staple cartridge assembly, a type of the staple cartridge assembly corresponding to the combination L/ is a gray staple cartridge assembly, a type of the staple cartridge assembly corresponding to the combination /H is a yellow staple cartridge assembly, a type of the staple cartridge assembly corresponding to the combination /L is a purple staple cartridge assembly, a type of the staple cartridge assembly corresponding to the combination // is a red staple cartridge assembly or no staple cartridge assembly.

It may be understood that shapes or sizes of staples placed in different colors of suture cartridge assemblies are different, such that the staple cartridge assemblies are suitable for suture of different thicknesses of patient's tissues.

**Table 1: correspondences between the combination of the first characteristic value and the second characteristic value and the type of the staple cartridge assembly**

| Corresponding figures | First characteristic value | Second characteristic value | Type of staple cartridge assembly |
|---|---|---|---|
| FIG. 6A | H | H | Black staple cartridge assembly |
| FIG. 6B | H | L | Green staple cartridge assembly |
| FIG. 6C | L | H | Blue staple cartridge assembly |
| FIG. 6D | L | L | White staple cartridge assembly |
| FIG. 6E | H | / | Gold staple cartridge assembly |
| FIG. 6F | L | / | Gray staple cartridge assembly |
| FIG. 6G | / | H | Yellow staple cartridge assembly |
| FIG. 6H | / | L | Purple staple cartridge assembly |
| FIG. 6I | / | / | Red staple cartridge assembly or no staple cartridge assembly |

It may be understood that the schematic curve views of the driving current shown in FIG. 6A to FIG. 6I and the correspondences shown in Table 1 are only an embodiment in which the type of the staple cartridge assembly 400 is determined according to the first characteristic value and the second characteristic value. The application further includes other embodiments with equivalent meanings, for example, the range within which the first characteristic value and the second characteristic value fall is divided more finely, such that each of the first characteristic value and the second characteristic value takes more values, for example, each of the first characteristic value and the second characteristic value takes 4, 5, 6 or more values, and there are more combinations of the first characteristic value and the second characteristic value correspondingly, therefore more types of staple cartridge assemblies may be identified.

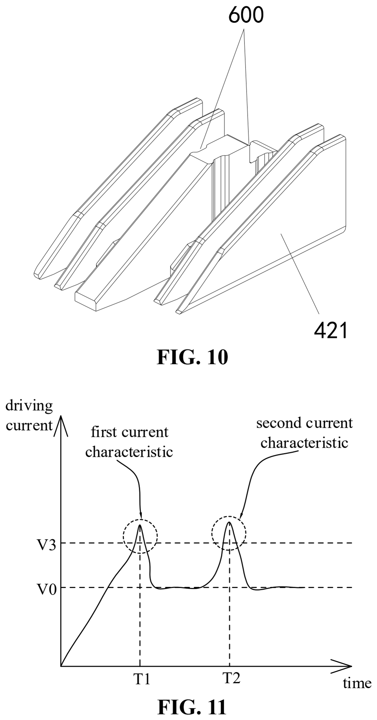

In an embodiment, with reference to FIG. 8, the adjustment member is a protrusion 500 arranged on the staple cartridge base 410, and the firing member 420 is provided with a groove 600 matched with the protrusion 500. Specifically, the firing member 420 includes a slider 421, and as shown in FIG. 9 and FIG. 10, the groove 600 of the firing member 420 is arranged in the slider 421.

In this embodiment, when the transmission mechanism 210 drives the firing member 420 to move to the first resistance adjustment position or the second resistance adjustment position, the groove 600 in the slider 421 may be matched and abutted with the protrusion 500 at the first resistance adjustment position or the second resistance adjustment position, thereby increasing the resistance received by the firing member 420. If the firing member 420 wants to continue moving forward, the firing member 420 needs to break away from cooperative abutment between the groove 600 in the slider 421 and the protrusion 500 on the staple cartridge base 410, therefore the driving current of the driving apparatus may increase, to provide a greater torque to drive the transmission mechanism 210 to move.

In an embodiment, the protrusion 500 may be arranged only at each of the first resistance adjustment position and the second resistance adjustment position of the staple cartridge base 410, to be used as the adjustment member, and it is unnecessary to arrange the groove 600 in the firing member 420.

In this embodiment, when the transmission mechanism 210 drives the firing member 420 to move to the first resistance adjustment position or the second resistance adjustment position, the protrusion 500 at the position may press a side surface of the firing member 420, thereby increasing a friction force received by the firing member 420, and achieving an effect of changing the resistance received by the firing member 420 when the firing member 420 passes through the position.

In an embodiment, with reference to FIG. 2 and FIG. 3, the firing member 420 includes a cutting knife 422 in addition to the slider 421, one end of the cutting knife 422 is connected to the slider 421, and the other end of the cutting knife 422 is connected to the transmission mechanism 210.

In this embodiment, the groove 600 of the firing member 420 may be arranged in the cutting knife 422 in addition to the slider 421. When the transmission mechanism 210 drives the firing member 420 to move to the first resistance adjustment position or the second resistance adjustment position, the groove 600 in the cutting knife 422 may be matched and abutted with the protrusion 500 at the first resistance adjustment position or the second resistance adjustment position, thereby increasing the resistance received by the firing member 420. If the firing member 420 wants to continue moving forward, the firing member 420 needs to break away from cooperative abutment between the groove 600 in the cutting knife 422 and the protrusion 500 on the staple cartridge base 410, therefore the driving current of the driving apparatus may increase, to provide a greater torque to drive the transmission mechanism 210 to move.

Further, in this embodiment, for different types of staple cartridge assemblies 400, at least one of the following conditions is met:
the protrusion 500 at the first resistance adjustment position is different in shape, length or thickness;
the protrusion 500 at the first resistance adjustment position matched with the groove 600 is different in number;
the protrusion 500 at the second resistance adjustment position is different in shape, length or thickness; or
the protrusion 500 at the second resistance adjustment position matched with the groove 600 is different in number.

It may be understood that for different types of staple cartridge assemblies 400, it only needs any one of the adjustment members at the first resistance adjustment position and the second resistance adjustment position to generate different resistances to the firing member 420, then different combinations of the first current characteristic and the second current characteristic may be extracted from the driving current of the driving apparatus. Therefore, when the protrusion 500 at the first resistance adjustment position is different in shape, length or thickness, the first current characteristic extracted from the driving current of the driving apparatus may be different. When the protrusion 500 at the first resistance adjustment position matched with the groove 600 is different in number, the first current characteristic extracted from the driving current of the driving apparatus may also be different. When the protrusion 500 at the second resistance adjustment position is different in shape, length or thickness, the second current characteristic extracted from the driving current of the driving apparatus may be different. When the protrusion 500 at the second resistance adjustment position matched with the groove 600 is different in number, the second current characteristic extracted from the driving current of the driving apparatus may also be different. It may be understood that various adjustment members generating different resistances as described above may be present simultaneously, such that there are more diverse combinations of the first current characteristic and the second current characteristic, therefore more types of staple cartridge assemblies 400 may be identified.

In another embodiment, the adjustment member is a groove arranged in the staple cartridge base 410, and the firing member 420 is provided with a protrusion matched with the groove. Specifically, the firing member 420 includes a slider 421, and the groove 600 of the firing member 420 is arranged in the protrusion.

In this embodiment, when the transmission mechanism 210 drives the firing member 420 to move to the first resistance adjustment position or the second resistance adjustment position, the protrusion on the slider 421 may be matched and abutted with the groove at the first resistance adjustment position or the second resistance adjustment position, thereby increasing the resistance received by the firing member 420. If the firing member 420 wants to continue moving forward, the firing member 420 needs to break away from cooperative abutment between the protrusion on the slider 421 and the groove in the staple cartridge base 410, therefore the driving current of the driving apparatus may increase, to provide a greater torque to drive the transmission mechanism 210 to move.

In an embodiment, with reference to FIG. 2 and FIG. 3, the firing member 420 includes a cutting knife 422 in addition to the slider 421, one end of the cutting knife 422 is connected to the slider 421, and the other end of the cutting knife 422 is connected to the transmission mechanism 210.

In this embodiment, the protrusion on the firing member 420 may be arranged on the cutting knife 422 in addition to the slider 421. When the transmission mechanism 210 drives the firing member 420 to move to the first resistance adjustment position or the second resistance adjustment position, the protrusion on the cutting knife 422 may be matched and abutted with the groove at the first resistance adjustment position or the second resistance adjustment position, thereby increasing the resistance received by the firing member 420. If the firing member 420 wants to continue moving forward, the firing member 420 needs to break away from cooperative abutment between the protrusion on the cutting knife 422 and the groove in the staple cartridge base 410, therefore the driving current of the driving apparatus may increase, to provide a greater torque to drive the transmission mechanism 210 to move.

In an embodiment, for different types of staple cartridge assemblies 400, at least one of the following conditions is met:
a distance between the first resistance adjustment position and the firing initial position 401 is different;
a distance between the second resistance adjustment position and the firing initial position 401 is different; or
a distance between the first resistance adjustment position and the second resistance adjustment position is different.

It may be understood that in the foregoing embodiments, different types of staple cartridge assemblies 400 are distinguished by different resistances generated to the firing member 420 at the first resistance adjustment position or the second resistance adjustment position, such that different magnitudes of the first current characteristic and the second current characteristic are extracted from the driving current of the driving apparatus.

In this embodiment, different types of staple cartridge assemblies 400 are distinguished by different distances between the first resistance adjustment position and the second resistance adjustment position, such that the first current characteristic and the second current characteristic extracted from the driving current of the driving apparatus are present at different time intervals.

In an embodiment, each of the first current characteristic and the second current characteristic includes multiple consecutive current values, the control apparatus configured to determine a type of the staple cartridge assembly 400 according to the first current characteristic and the second current characteristic, includes any one of: the control apparatus configured to:
determine the type of the staple cartridge assembly 400 according to a time interval between a current peak value in the first current characteristic and a current peak value in the second current characteristic; or
determine the type of the staple cartridge assembly 400 according to a first time period T1 corresponding to the firing member 420 starting to move until reaching a current peak value in the first current characteristic, and a second time period T2 corresponding to the firing member 420 starting to move until reaching a current peak value in the second current characteristic.

In this embodiment, with reference to FIG. 11, when the distance between the first resistance adjustment position and the firing initial position 401 is different, the first time period T1 corresponding to the firing member 420 starting to move until reaching a current peak value in the first current characteristic is different in the first current characteristic extracted from the driving current of the driving apparatus. Similarly, when the distance between the second resistance adjustment position and the firing initial position 401 is different, the second time period T2 corresponding to the firing member 420 starting to move until reaching a current peak value in the second current characteristic is different in the second current characteristic extracted from the driving current of the driving apparatus. In the above two cases, the distance between the first resistance adjustment position and the second resistance adjustment position is different, resulting in that the time interval between the current peak value in the first current characteristic and the current peak value in the second current characteristic is different, that is, a time interval between T1 and T2 in FIG. 11 is different, then the control apparatus may determine the type of the staple cartridge assembly 400 according to difference in the time interval.

According to another aspect, an embodiment of the application provides a surgical instrument. With reference to FIG. 1, the surgical instrument includes a handle assembly 100, a tube assembly 200, a jaw assembly 300, and a staple cartridge assembly 400.

The handle assembly 100 includes a driving apparatus and a control apparatus. The driving apparatus and the control apparatus are arranged inside a housing of the handle assembly 100, and thus the driving apparatus and the control apparatus are not shown in FIG. 1.

An end of the tube assembly 200 is connected to the handle assembly 100, the tube assembly 200 includes a transmission mechanism 210 connected to the driving apparatus. Specifically, the transmission mechanism 210 is arranged inside the tube assembly 200, and thus the transmission mechanism 210 is not shown in FIG. 1.

Another end of the tube assembly 200 is connected to the jaw assembly 300, the jaw assembly 300 includes a first jaw 310 and a second jaw 320, the first jaw 310 is movably connected to the second jaw 320, the first jaw 310 and the second jaw 320 are capable of moving relatively to each other to implement closure and opening, a mounting cavity is formed between the first jaw 310 and the second jaw 320. The first jaw 310 and the second jaw 320 shown in FIG. 1 are in an open state, and the first jaw 310 and the second jaw 320 shown in FIG. 2 are in a closed state.

The staple cartridge assembly 400 is mounted in the mounting cavity. As shown in FIG. 2 and FIG. 3, the staple cartridge assembly 400 includes a staple cartridge base 410 and a firing member 420, the firing member 420 is arranged at the staple cartridge base 410, the staple cartridge base 410 is provided with a mounting slot 411 where staples are mounted, a firing initial position 401 away from the mounting slot 411 and a firing end position 402 close to the mounting slot 411. With reference to FIG. 2, after the staple cartridge assembly 400 is mounted into the mounting cavity in the jaw assembly 300, the transmission mechanism 210 is cooperatively connected to the firing member 420 of the staple cartridge assembly 400, such that the transmission mechanism 210 is capable of driving the firing member 420 to move from the firing initial position 401 to the firing end position 402, to complete a process of firing the staple cartridge assembly 400.

At least one resistance adjustment position is provided between the firing initial position 401 and the firing end position 402, an adjustment member is arranged at the resistance adjustment position and is configured to change a resistance received by the firing member 420 when the firing member 420 passes through the resistance adjustment position. It may be understood that in the process of firing the staple cartridge assembly 400, that is, in a process of the transmission mechanism 210 driving the firing member 420 to move from the firing initial position 401 to the firing end position 402, the firing member 420 may pass through the at least one resistance adjustment position as provided, and the adjustment member arranged at the at least one resistance adjustment position may change the resistance received by the firing member 420 when the firing member 420 passes through the at least one resistance adjustment position. The resistance encountered by the firing member 420 in the firing process may be represented by a variation of a driving current of the driving apparatus, such that corresponding current characteristics are present in the driving current of the driving apparatus.

The control apparatus is configured to:
acquire a driving current of the driving apparatus, and extract, from the driving current, current characteristic(s) corresponding to the at least one resistance adjustment position; and
determine a type of the staple cartridge assembly 400 according to the current characteristic(s), or determine a driving mode applied by the driving apparatus to the transmission mechanism 210 according to the current characteristic(s).

It may be understood that the driving current of the driving apparatus should be a relatively stable current with little fluctuation when the firing member 420 does not encounter any adjustment member, and at this time, the driving apparatus only needs to overcome a friction force between the firing member 420 and the staple cartridge base 410 to drive the firing member 420. However, the adjustment member arranged at the at least one resistance adjustment position may increase the resistance received by the firing member 420 when the firing member 420 passes through the adjustment member at the at least one resistance adjustment position, and the driving apparatus needs a greater driving current to generate a torque enough to drive the transmission mechanism 210, and then the transmission mechanism 210 drives the firing member 420 to go away from the resistance adjustment position to continue moving forward. After acquiring the driving current of the driving apparatus, the control apparatus may extract, from the driving current of the driving apparatus, at least one current characteristic that is greater than currents at other positions. The type of the staple cartridge assembly 400 is determined according to current characteristic(s) corresponding to the at least one resistance adjustment position. In this way, it is unnecessary to provide electrical components and electrical wiring on the staple cartridge assembly 400, to avoid abnormal identification of the staple cartridge assembly 400 due to blockage of the electrical circuit, such that it may implement identification of the staple cartridge assembly 400 safely and reliably. The driving mode applied by the driving apparatus to the transmission mechanism 210 is determined according to the current characteristic(s) corresponding to the at least one resistance adjustment position, to avoid driving the driving apparatus of the staple cartridge assembly 400 by using an inappropriate driving mode, such that it may ensure the suture effect of the patient's tissue.

In an embodiment, for different types of staple cartridge assemblies 400, resistance generated to the firing member 420 at at least one resistance adjustment position is different.

In this embodiment, exemplarily, assuming that the driving current of the driving apparatus usually fluctuates around 0.5A when the firing member 420 does not encounter any adjustment member; and when the firing member 420 passes through the adjustment member, it needs to overcome resistance brought by the adjustment member, the driving current of the driving apparatus may increase to be greater than 0.8A, and then at least one convex region may be present in a curve of the driving current of the driving apparatus and may be current characteristic(s) corresponding to the at least one resistance adjustment position, and each current characteristic is formed of multiple consecutive current values greater than 0.8A. If the adjustment member at the resistance adjustment position generates a small resistance to the firing member 420, multiple current values in a corresponding current characteristic are greater than 0.8A but less than 1.2A; if the adjustment member at the resistance adjustment position generates a large resistance to the firing member 420, multiple current values in a corresponding current characteristic are greater than 1.2A. Therefore, in this embodiment, corresponding characteristic values may be determined according to a range within which a current peak value or an average current value in the at least one current characteristic falls, and the type of the staple cartridge assembly 400 may be determined according to a combination of the characteristic values.

It may be understood that implementing the adjustment member at the resistance adjustment position by using what kind of structure specifically, and how to implement different resistances generated to the firing member 420, may be described with reference to introductions and descriptions of the foregoing embodiments and will not be repeated and elaborated here.

In an embodiment, for different types of staple cartridge assemblies 400, the resistance adjustment position is different in number.

The control apparatus configured to determine a type of the staple cartridge assembly 400 according to the current characteristic(s), includes: the control apparatus configured to:
determine the type of the staple cartridge assembly 400 according to the number of the current characteristic(s).

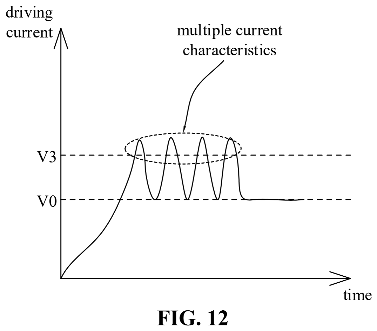

In this embodiment, with reference to FIG. 12, for different types of staple cartridge assemblies 400, the number of resistance adjustment positions is different, then the number of current characteristics extracted from the driving current of the driving apparatus is different, therefore the type of the staple cartridge assembly 400 may be determined according to the number of the current characteristic(s). It may be understood that in this embodiment, it may not need to pay attention to magnitudes of the current characteristics, instead, it only needs to pay attention to whether the current characteristics are present, and the adjustment member arranged at each resistance adjustment position may be the same.

According to another aspect, an embodiment of the application provides a surgical instrument. With reference to FIG. 1, the surgical instrument includes a handle assembly 100, a tube assembly 200, a jaw assembly 300, and a staple cartridge assembly 400.

The handle assembly 100 includes a driving apparatus and a control apparatus. The driving apparatus and the control apparatus are arranged inside a housing of the handle assembly 100, and thus the driving apparatus and the control apparatus are not shown in FIG. 1.

An end of the tube assembly 200 is connected to the handle assembly 100, the tube assembly 200 includes a transmission mechanism 210 connected to the driving apparatus. The transmission mechanism 210 is arranged inside the tube assembly 200, and thus the transmission mechanism 210 is not shown in FIG. 1.

Another end of the tube assembly 200 is connected to the jaw assembly 300, the jaw assembly 300 includes a first jaw 310 and a second jaw 320, the first jaw 310 is movably connected to the second jaw 320, the first jaw 310 and the second jaw 320 are capable of moving relatively to each other to implement closure and opening, a mounting cavity is formed between the first jaw 310 and the second jaw 320. The first jaw 310 and the second jaw 320 shown in FIG. 1 are in an open state, and the first jaw 310 and the second jaw 320 shown in FIG. 2 are in a closed state.

The staple cartridge assembly 400 is mounted in the mounting cavity. As shown in FIG. 2 and FIG. 3, the staple cartridge assembly 400 includes a staple cartridge base 410 and a firing member 420, the firing member 420 is arranged at the staple cartridge base 410, the staple cartridge base 410 is provided with a mounting slot 411 where staples are mounted, a firing initial position 401 away from the mounting slot 411 and a firing end position 402 close to the mounting slot 411. With reference to FIG. 2, after the staple cartridge assembly 400 is mounted into the mounting cavity in the jaw assembly 300, the transmission mechanism 210 is cooperatively connected to the firing member 420 of the staple cartridge assembly 400, such that the transmission mechanism 210 is capable of driving the firing member 420 to move from the firing initial position 401 to the firing end position 402, to complete a process of firing the staple cartridge assembly 400.

A first resistance adjustment position and a second resistance adjustment position are provided between the firing initial position 401 and the firing end position 402, and an adjustment member is arranged at each of the first resistance adjustment position and the second resistance adjustment position and is configured to change a resistance received by the transmission mechanism 210 when the transmission mechanism 210 passes through each of the first resistance adjustment position and the second resistance adjustment position. It may be understood that in the process of firing the staple cartridge assembly 400, that is, in a process of the transmission mechanism 210 driving the firing member 420 to move from the firing initial position 401 to the firing end position 402, the transmission mechanism 210 may pass through the first resistance adjustment position and the second resistance adjustment position, and adjustment members arranged at the two resistance adjustment positions respectively may change resistances received by the transmission mechanism 210 when the transmission mechanism 210 passes through the two resistance adjustment positions respectively. The resistance encountered by the transmission mechanism 210 in the firing process may be represented by a variation of a driving current of the driving apparatus, such that corresponding current characteristics are present in the driving current of the driving apparatus.

The control apparatus is configured to:
acquire a driving current of the driving apparatus, and extract, from the driving current, a first current characteristic corresponding to the first resistance adjustment position and a second current characteristic corresponding to the second resistance adjustment position; and
determine a type of the staple cartridge assembly 400 according to the first current characteristic and the second current characteristic, or determine a driving mode applied by the driving apparatus to the transmission mechanism 210 according to the first current characteristic and the second current characteristic.

It may be understood that the driving current of the driving apparatus should be a relatively stable current with little fluctuation when the transmission mechanism 210 does not encounter any adjustment member, and at this time, the driving apparatus only needs to overcome a friction force between the firing member 420 and the staple cartridge base 410 to drive the firing member 420. However, adjustment members arranged at the first resistance adjustment position and the second resistance adjustment position respectively may increase resistances received by the transmission mechanism 210 when the transmission mechanism 210 passes through the adjustment members at the first resistance adjustment position and the second resistance adjustment position respectively, and the driving apparatus needs a greater driving current to generate a torque enough to drive the transmission mechanism 210 away from the first resistance adjustment position and the second resistance adjustment position to continue moving forward. After the driving current of the driving apparatus is acquired, the first current characteristic and the second current characteristic that are greater than currents at other positions may be extracted from the driving current of the driving apparatus. The type of the staple cartridge assembly 400 is determined according to a difference between the current characteristics corresponding to the two resistance adjustment positions respectively. In this way, it is unnecessary to provide electrical components and electrical wiring on the staple cartridge assembly 400, to avoid abnormal identification of the staple cartridge assembly 400 due to blockage of the electrical circuit, such that it may implement identification of the staple cartridge assembly 400 safely and reliably. The driving mode applied by the driving apparatus to the transmission mechanism 210 is determined according to the difference between the current characteristics corresponding to the two resistance adjustment positions respectively, to avoid driving the driving apparatus of the staple cartridge assembly 400 by using an inappropriate driving mode, such that it may ensure the suture effect of the patient's tissue.

According to another aspect, an embodiment of the application provides a surgical instrument. With reference to FIG. 1, the surgical instrument includes a handle assembly 100, a tube assembly 200, a jaw assembly 300, and a staple cartridge assembly 400.

The handle assembly 100 includes a driving apparatus and a control apparatus. The driving apparatus and the control apparatus are arranged inside a housing of the handle assembly 100, and thus the driving apparatus and the control apparatus are not shown in FIG. 1.

An end of the tube assembly 200 is connected to the handle assembly 100, the tube assembly 200 includes a transmission mechanism 210 connected to the driving apparatus. Specifically, the transmission mechanism 210 is arranged inside the tube assembly 200, and thus the transmission mechanism 210 is not shown in FIG. 1.

Another end of the tube assembly 200 is connected to the jaw assembly 300, the jaw assembly 300 includes a first jaw 310 and a second jaw 320, the first jaw 310 is movably connected to the second jaw 320, the first jaw 310 and the second jaw 320 are capable of moving relatively to each other to implement closure and opening, a mounting cavity is formed between the first jaw 310 and the second jaw 320. The first jaw 310 and the second jaw 320 shown in FIG. 1 are in an open state, and the first jaw 310 and the second jaw 320 shown in FIG. 2 are in a closed state.

The staple cartridge assembly 400 is mounted in the mounting cavity. As shown in FIG. 2 and FIG. 3, the staple cartridge assembly 400 includes a staple cartridge base 410 and a firing member 420, the firing member 420 is arranged at the staple cartridge base 410, the staple cartridge base 410 is provided with a mounting slot 411 where staples are mounted, a firing initial position 401 away from the mounting slot 411 and a firing end position 402 close to the mounting slot 411. With reference to FIG. 2, after the staple cartridge assembly 400 is mounted into the mounting cavity in the jaw assembly 300, the transmission mechanism 210 is cooperatively connected to the firing member 420 of the staple cartridge assembly 400, such that the transmission mechanism 210 is capable of driving the firing member 420 to move from the firing initial position 401 to the firing end position 402, to complete a process of firing the staple cartridge assembly 400.

At least one resistance adjustment position is provided between the firing initial position 401 and the firing end position 402, an adjustment member is arranged at the resistance adjustment position and is configured to change a resistance received by the transmission mechanism 210 when the transmission mechanism 210 passes through the resistance adjustment position. It may be understood that in the process of firing the staple cartridge assembly 400, that is, in a process of the transmission mechanism 210 driving the firing member 420 to move from the firing initial position 401 to the firing end position 402, the transmission mechanism 210 may pass through the at least one resistance adjustment position as provided, and the adjustment member arranged at the at least one resistance adjustment position may change the resistance received by the transmission mechanism 210 when the transmission mechanism 210 passes through the at least one resistance adjustment position. The resistance encountered by the firing member 420 in the firing process may be represented by a variation of a driving current of the driving apparatus, such that corresponding current characteristics are present in the driving current of the driving apparatus.

The control apparatus is configured to:
acquire a driving current of the driving apparatus, and extract, from the driving current, current characteristic(s) corresponding to the at least one resistance adjustment position; and
determine a type of the staple cartridge assembly 400 according to the current characteristic(s), or determine a driving mode applied by the driving apparatus to the transmission mechanism 210 according to the current characteristic(s).

It may be understood that the driving current of the driving apparatus should be a relatively stable current with little fluctuation when the transmission mechanism 210 does not encounter any adjustment member, and at this time, the driving apparatus only needs to overcome a friction force between the firing member 420 and the staple cartridge base 410 to drive the firing member 420. However, the adjustment member arranged at the at least one resistance adjustment position may increase the resistance received by the transmission mechanism 210 when the transmission mechanism 210 passes through the adjustment member at the at least one resistance adjustment position, and the driving apparatus needs a greater driving current to generate a torque enough to drive the transmission mechanism 210 away from the resistance adjustment position to continue moving forward. After acquiring the driving current of the driving apparatus, the control apparatus may extract, from the driving current of the driving apparatus, at least one current characteristic that is greater than currents at other positions. The type of the staple cartridge assembly 400 is determined according to current characteristic(s) corresponding to the at least one resistance adjustment position. In this way, it is unnecessary to provide electrical components and electrical wiring on the staple cartridge assembly 400, to avoid abnormal identification of the staple cartridge assembly 400 due to blockage of the electrical circuit, such that it may implement identification of the staple cartridge assembly 400 safely and reliably. The driving mode applied by the driving apparatus to the transmission mechanism 210 is determined according to the current characteristic(s) corresponding to the at least one resistance adjustment position, to avoid driving the driving apparatus of the staple cartridge assembly 400 by using an inappropriate driving mode, such that it may ensure the suture effect of the patient's tissue.

It should also be understood that various implementations provided in the embodiments of the application may be arbitrarily combined to achieve different technical effects.

The above descriptions are specific descriptions of preferred implementations of the application; however, the application is not limited to the above implementations. Technicians familiar with this field may also make various equivalent modifications or replacements in a shared condition of not violating the spirit of the application, and these equivalent modifications or replacements fall within the scope defined by the claims of the application.

## Claims

1. A surgical instrument, comprising:
a handle assembly (100), comprising a driving apparatus and a control apparatus;
a tube assembly (200), of which an end is connected to the handle assembly (100), the tube assembly (200) comprising a transmission mechanism (210) connected to the driving apparatus;
a jaw assembly (300), to which another end of the tube assembly (200) is connected, the jaw assembly (300) comprising a first jaw (310) and a second jaw (320), the first jaw (310) movably connected to the second jaw (320), the first jaw (310) and the second jaw (320) being capable of moving relatively to each other to implement closure and opening, a mounting cavity being formed between the first jaw (310) and the second jaw (320); and
a staple cartridge assembly (400), mounted in the mounting cavity and comprising a staple cartridge base (410) and a firing member (420) arranged at the staple cartridge base (410), the staple cartridge base (410) being provided with a mounting slot (411) where staples are mounted, a firing initial position (401) being away from the mounting slot (411) and a firing end position (402) being close to the mounting slot (411), the transmission mechanism (210) being capable of driving the firing member (420) to move from the firing initial position (401) to the firing end position (402), a first resistance adjustment position and a second resistance adjustment position being provided between the firing initial position (401) and the firing end position (402), an adjustment member being arranged at each of the first resistance adjustment position and the second resistance adjustment position and configured to change a resistance received by the firing member (420) when the firing member (420) passes through each of the first resistance adjustment position and the second resistance adjustment position,
wherein the control apparatus is configured to:
acquire a driving current of the driving apparatus, and extract, from the driving current, a first current characteristic corresponding to the first resistance adjustment position and a second current characteristic corresponding to the second resistance adjustment position; and
determine a type of the staple cartridge assembly (400) according to the first current characteristic and the second current characteristic, or determine a driving mode applied by the driving apparatus to the transmission mechanism (210) according to the first current characteristic and the second current characteristic.

2. The surgical instrument of claim 1, wherein for different types of staple cartridge assemblies (400), resistances generated to the firing member (420) at the first resistance adjustment position and/or the second resistance adjustment position are different; each of the first current characteristic and the second current characteristic comprises a plurality of consecutive current values,
the control apparatus configured to determine a type of the staple cartridge assembly (400) according to the first current characteristic and the second current characteristic, comprises: the control apparatus configured to:
determine a first characteristic value according to a range within which a current peak value or an average current value in the first current characteristic falls;
determine a second characteristic value according to a range within which a current peak value or an average current value in the second current characteristic falls; and
determine the type of the staple cartridge assembly (400) according to a combination of the first characteristic value and the second characteristic value.

3. The surgical instrument of claim 1, wherein the adjustment member is a protrusion (500) arranged on the staple cartridge base (410).

4. The surgical instrument of claim 3, wherein the firing member (420) is provided with a groove (600) matched with the protrusion (500).

5. The surgical instrument of claim 3, wherein for different types of staple cartridge assemblies (400), at least one of the following conditions is met:
the protrusion (500) at the first resistance adjustment position is different in shape, length or thickness; or
the protrusion (500) at the second resistance adjustment position is different in shape, length or thickness.

6. The surgical instrument of claim 1, wherein the adjustment member is a groove arranged in the staple cartridge base (410), and the firing member (420) is provided with a protrusion matched with the groove.

7. The surgical instrument of claim 4, wherein for different types of staple cartridge assemblies (400), at least one of the following conditions is met:
the protrusion (500) at the first resistance adjustment position is different in shape, length or thickness;
the protrusion (500) at the first resistance adjustment position matched with the groove (600) is different in number;
the protrusion (500) at the second resistance adjustment position is different in shape, length or thickness; or
the protrusion (500) at the second resistance adjustment position matched with the groove (600) is different in number.

8. The surgical instrument of claim 4, wherein the firing member (420) comprises a slider (421) and a cutting knife (422), one end of the cutting knife (422) is connected to the slider (421), and the other end of the cutting knife (422) is connected to the transmission mechanism (210); the groove (600) is arranged in the slider (421) or the cutting knife (422).

9. The surgical instrument of claim 6, wherein the firing member (420) comprises a slider (421) and a cutting knife (422), one end of the cutting knife (422) is connected to the slider (421), and the other end of the cutting knife (422) is connected to the transmission mechanism (210); the protrusion is arranged on the slider (421) or the cutting knife (422).

10. The surgical instrument of claim 1, wherein for different types of staple cartridge assemblies (400), at least one of the following conditions is met:
a distance between the first resistance adjustment position and the firing initial position (401) is different;
a distance between the second resistance adjustment position and the firing initial position (401) is different; or
a distance between the first resistance adjustment position and the second resistance adjustment position is different.

11. The surgical instrument of claim 10, wherein each of the first current characteristic and the second current characteristic comprises a plurality of consecutive current values, the control apparatus configured to determine a type of the staple cartridge assembly (400) according to the first current characteristic and the second current characteristic, comprises any one of: the control apparatus configured to:
determine the type of the staple cartridge assembly (400) according to a time interval between a current peak value in the first current characteristic and a current peak value in the second current characteristic; or
determine the type of the staple cartridge assembly (400) according to a first time period corresponding to the firing member (420) starting to move until reaching a current peak value in the first current characteristic, and a second time period corresponding to the firing member (420) starting to move until reaching a current peak value in the second current characteristic.

12. A surgical instrument, comprising:
a handle assembly (100), comprising a driving apparatus and a control apparatus;
a tube assembly (200), of which an end is connected to the handle assembly (100), the tube assembly (200) comprising a transmission mechanism (210) connected to the driving apparatus;
a jaw assembly (300), to which another end of the tube assembly (200) is connected, the jaw assembly (300) comprising a first jaw (310) and a second jaw (320), the first jaw (310) movably connected to the second jaw (320), the first jaw (310) and the second jaw (320) being capable of moving relatively to each other to implement closure and opening, a mounting cavity being formed between the first jaw (310) and the second jaw (320); and
a staple cartridge assembly (400), mounted in the mounting cavity and comprising a staple cartridge base (410) and a firing member (420) arranged at the staple cartridge base (410), the staple cartridge base (410) provided with a mounting slot (411) where staples are mounted, a firing initial position (401) away from the mounting slot (411) and a firing end position (402) close to the mounting slot (411), the transmission mechanism (210) being capable of driving the firing member (420) to move from the firing initial position (401) to the firing end position (402), at least one resistance adjustment position being provided between the firing initial position (401) and the firing end position (402), an adjustment member being arranged at the resistance adjustment position and configured to change a resistance received by the firing member (420) when the firing member (420) passes through the resistance adjustment position,
wherein the control apparatus is configured to:
acquire a driving current of the driving apparatus, and extract, from the driving current, current characteristic(s) corresponding to the at least one resistance adjustment position; and
determine a type of the staple cartridge assembly (400) according to the current characteristic(s), or determine a driving mode applied by the driving apparatus to the transmission mechanism (210) according to the current characteristic(s).

13. The surgical instrument of claim 12, wherein for different types of staple cartridge assemblies (400), the resistance adjustment position is different in number,
the control apparatus configured to determine a type of the staple cartridge assembly (400) according to the current characteristic(s), comprises: the control apparatus configured to:
determine the type of the staple cartridge assembly (400) according to the number of the current characteristic(s).

14. A surgical instrument, comprising:
a handle assembly (100), comprising a driving apparatus and a control apparatus;
a tube assembly (200), of which an end is connected to the handle assembly (100), the tube assembly (200) comprising a transmission mechanism (210) connected to the driving apparatus;
a jaw assembly (300), to which another end of the tube assembly (200) is connected, the jaw assembly (300) comprising a first jaw (310) and a second jaw (320), the first jaw (310) movably connected to the second jaw (320), the first jaw (310) and the second jaw (320) being capable of moving relatively to each other to implement closure and opening, a mounting cavity being formed between the first jaw (310) and the second jaw (320); and
a staple cartridge assembly (400), mounted in the mounting cavity and comprising a staple cartridge base (410) and a firing member (420) arranged at the staple cartridge base (410), the staple cartridge base (410) provided with a mounting slot (411) where staples are mounted, a firing initial position (401) away from the mounting slot (411) and a firing end position (402) close to the mounting slot (411), the transmission mechanism (210) being capable of driving the firing member (420) to move from the firing initial position (401) to the firing end position (402), a first resistance adjustment position and a second resistance adjustment position being provided between the firing initial position (401) and the firing end position (402), an adjustment member being arranged at each of the first resistance adjustment position and the second resistance adjustment position and configured to change a resistance received by the transmission mechanism (210) when the transmission mechanism (210) passes through each of the first resistance adjustment position and the second resistance adjustment position,
wherein the control apparatus is configured to:
acquire a driving current of the driving apparatus, and extract, from the driving current, a first current characteristic corresponding to the first resistance adjustment position and a second current characteristic corresponding to the second resistance adjustment position; and
determine a type of the staple cartridge assembly (400) according to the first current characteristic and the second current characteristic, or determine a driving mode applied by the driving apparatus to the transmission mechanism (210) according to the first current characteristic and the second current characteristic.

15. A surgical instrument, comprising:
a handle assembly (100), comprising a driving apparatus and a control apparatus;
a tube assembly (200), of which an end is connected to the handle assembly (100), the tube assembly (200) comprising a transmission mechanism (210) connected to the driving apparatus;
a jaw assembly (300), to which another end of the tube assembly (200) is connected, the jaw assembly (300) comprising a first jaw (310) and a second jaw (320), the first jaw (310) movably connected to the second jaw (320), the first jaw (310) and the second jaw (320) being capable of moving relatively to each other to implement closure and opening, a mounting cavity being formed between the first jaw (310) and the second jaw (320); and
a staple cartridge assembly (400), mounted in the mounting cavity and comprising a staple cartridge base (410) and a firing member (420) arranged at the staple cartridge base (410), the staple cartridge base (410) provided with a mounting slot (411) where staples are mounted, a firing initial position (401) away from the mounting slot (411) and a firing end position (402) close to the mounting slot (411), the transmission mechanism (210) being capable of driving the firing member (420) to move from the firing initial position (401) to the firing end position (402), at least one resistance adjustment position being provided between the firing initial position (401) and the firing end position (402), an adjustment member being arranged at the resistance adjustment position and configured to change a resistance received by the transmission mechanism (210) when the transmission mechanism (210) passes through the resistance adjustment position,
wherein the control apparatus is configured to:
acquire a driving current of the driving apparatus, and extract, from the driving current, current characteristic(s) corresponding to the at least one resistance adjustment position; and
determine a type of the staple cartridge assembly (400) according to the current characteristic(s), or determine a driving mode applied by the driving apparatus to the transmission mechanism (210) according to the current characteristic(s).
